(19) Europäisches Patentamt
European Patent Office
Office européen des brevets

(11) **EP 2 763 067 B1**

(12) **EUROPEAN PATENT SPECIFICATION**

(45) Date of publication and mention
of the grant of the patent:
**24.04.2019 Bulletin 2019/17**

(51) Int Cl.:
***G16H 50/50*** *(2018.01)*

(21) Application number: **14152473.6**

(22) Date of filing: **24.01.2014**

(54) **Biological simulation program, biological simulation method, and biological simulation device**

Biologisches Simulationsprogramm, biologisches Simulationsverfahren und biologische Simulationsvorrichtung

Programme de simulation biologique, procédé de simulation biologique et dispositif de simulation biologique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2013 JP 2013017680**

(43) Date of publication of application:
**06.08.2014 Bulletin 2014/32**

(73) Proprietors:
• **FUJITSU LIMITED**
**Kawasaki-shi,**
**Kanagawa 211-8588 (JP)**
• **The University of Tokyo**
**Bunkyo-ku,**
**Tokyo 113-8654 (JP)**

(72) Inventors:
• **Washio, Takumi**
**Bunkyo-ku, Tokyo 113-8654 (JP)**
• **Hisada, Toshiaki**
**Bunkyo-ku, Tokyo 113-8654 (JP)**
• **Sugiura, Seiryo**
**Bunkyo-ku, Tokyo 113-8654 (JP)**
• **Okada, Jun-ichi**
**Bunkyo-ku, Tokyo 113-8654 (JP)**
• **Takahashi, Akihito**
**Bunkyo-ku, Tokyo 113-8654 (JP)**
• **Yoneda, Kazunori**
**Kawasaki-shi, Kanagawa 211-8588 (JP)**
• **Matsunaga, Hiroyuki**
**Fukuoka-shi, Fukuoka 814-8589 (JP)**

(74) Representative: **Haseltine Lake LLP**
**Lincoln House, 5th Floor**
**300 High Holborn**
**London WC1V 7JH (GB)**

(56) References cited:
**GB-A- 2 441 078       US-A1- 2007 192 075**
**US-A1- 2010 318 326**

• **A. HOSOI; T. WASHIO; J. OKADA; Y. KADOOKA; K. NAKAJIMA; T. HISADA: "A multi-scale heart simulation on massively parallel computers", ACM/IEEE SUPERCOMPUTING CONFERENCE (SC10, 2010, XP002734139,**

**Description**

FIELD

The embodiments discussed herein are directed to a biological simulation program, a biological simulation method, and a biological simulation device.

BACKGROUND

Huge knowledge has been obtained about scales of organs, cells, and materials contained in a cell in a biological body with advancement of medical measurement techniques. It is, however, difficult for even experts to understand and predict relations among the scales of the organs, the cells, and the materials contained in a cell; for example, influences of behaviors of the cells and the organs on each other. For example, as for the heart, there are cardiac diseases of which pathological mechanisms have not been explained although responsible genes have been identified by global genetic analysis, such as familial hypertrophic cardiomyopathy.

For coping with this, there are techniques of simulating influences of the behaviors of the cells and the organs on each other. Examples of the techniques include a technique of simulating behaviors of a cardiomyocyte model and a heart model.

For example, see A. Hosoi, T. Washio, J. Okada, Y. Kadooka, K. Nakajima and T. Hisada, A multi-scale heart simulation on massively parallel computers, ACM/IEEE Supercomputing Conference (SC10), 2010.

In the above-mentioned technique of simulating the behaviors of the cardiomyocyte model and the heart model, simulation is performed on a structure contained in the cardiomyocyte, for example, a sarcomere, using an average model. That is, in the technique, the simulation is performed on a small structure contained in the cardiomyocyte, such as the sarcomere, using the average model. This raises a problem in that the simulation result lacks accuracy.

Accordingly, it is an object in one aspect of an embodiment of the invention to obtain an accurate simulation result.

US2007/192075 discloses an organism simulation device that is provided with two or more different simulator parts that calculate the behavior of organism structural elements, a data output part that visually outputs simulation results, and a simulation controller that controls the transfer of data between the two or more different simulator parts and the data output part based on simulation scenario information, which is information on the flow of data and the operation sequence.

SUMMARY

According to an aspect of the present invention, there is provided a biological simulation program according to claim 1.

According to another aspect of the present invention, there is provided a biological simulation device according to claim 2.

BRIEF DESCRIPTION OF DRAWINGS

FIG. 1 is a diagram illustrating an example of a functional configuration of a biological simulation device according to a first embodiment;
FIG. 2 is a view illustrating examples of macro models, a meso model, and a micro model;
FIG. 3 is a diagram illustrating an example of condition data;
FIG. 4 is a flowchart illustrating procedures of biological simulation processing in the first embodiment;
FIG. 5 is a flowchart illustrating procedures of simulation processing in the first embodiment;
FIG. 6A is a view illustrating an example of a simulation result of each model at a time step that is displayed on a display unit;
FIG. 6B is a view illustrating an example of a simulation result of each model at a time step that is displayed on the display unit;
FIG. 6C is a view illustrating an example of a simulation result of each model at a time step that is displayed on the display unit;
FIG. 7 is a time chart illustrating an example of execution timings of pieces of processing by a macro processor and a meso-micro processor in the first embodiment;
FIG. 8 is a diagram illustrating an example of a functional configuration of a biological simulation device according to a second embodiment;

FIG. 9 is a flowchart illustrating procedures of simulation processing in the second embodiment;
FIG. 10 is a time chart illustrating an example of execution timings of pieces of processing by a macro processor and a meso-micro processor in the second embodiment; and
FIG. 11 is a diagram illustrating a computer that executes a biological simulation program.

DESCRIPTION OF EMBODIMENTS

Preferred embodiments of the present invention will be explained with reference to accompanying drawings. Note that the embodiments do not limit disclosed techniques.

[a] First Embodiment

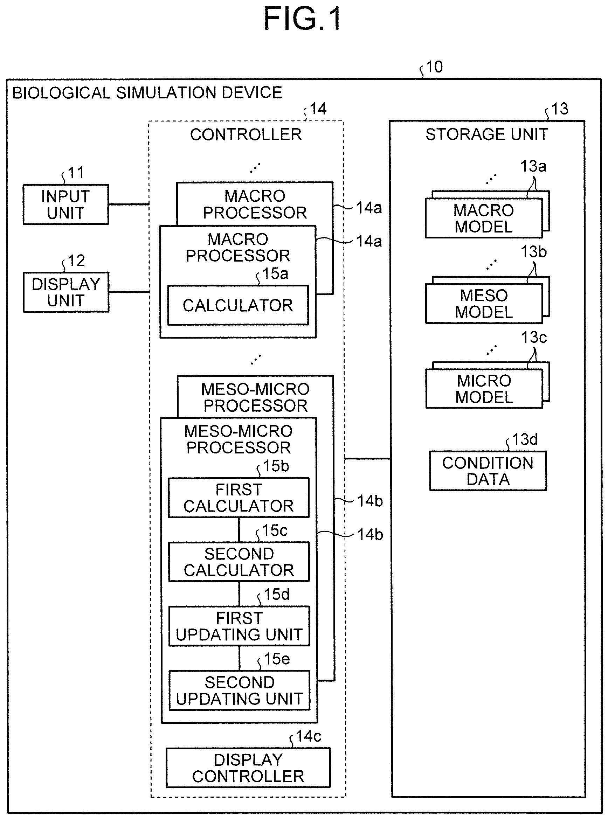

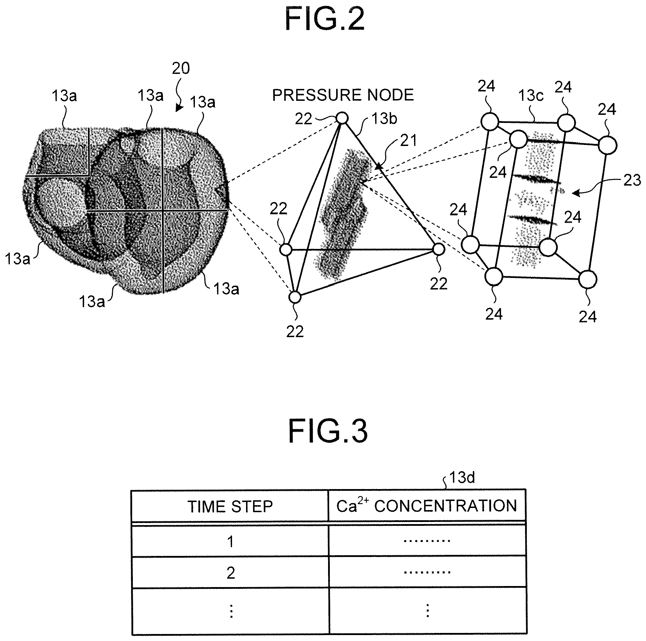

The following describes a biological simulation device according to a first embodiment. FIG. 1 is a diagram illustrating an example of a functional configuration of the biological simulation device in the first embodiment. This biological simulation device 10 includes an input unit 11, a display unit 12, a storage unit 13, and a controller 14.
The input unit 11 inputs various kinds of information to the controller 14. For example, upon receiving an instruction to execute biological simulation processing, which will be described later, from a user, the input unit 11 inputs the received instruction to the controller 14. The input unit 11 is, for example, a keyboard or a mouse.
The display unit 12 displays various kinds of information. For example, the display unit 12 displays a simulation result under control by a display controller 14c, which will be described later.
The storage unit 13 stores therein various types of programs that are executed by the controller 14. The storage unit 13 stores therein macro models 13a, meso models 13b, micro models 13c, and condition data 13d.
The macro models 13a are models obtained by dividing a model of the entire heart into a plurality of (for example, 10) portions. Note that the heart is an example of an organ of a biological body. The meso models 13b are models of the cardiomyocytes. Note that the cardiomyocyte is an example of a cell. The micro models 13c are models of sarcomeres contained in the cardiomyocyte. Note that the sarcomere is an example of a material contained in the cell. FIG. 2 is a view illustrating examples of the macro models, the meso model, and the micro model. The example of FIG. 2 illustrates the case where a model 20 of the entire heart is divided into a plurality of macro models 13a. A cardiac portion indicated by each of the macro models 13a contains a plurality of cardiomyocytes 21. The meso models 13b are models of the respective cardiomyocytes 21 contained in the macro model 13a. In the example of FIG. 2, each of the meso models 13b is a model having a shape of regular tetrahedron and has four node points 22. The cardiomyocytes indicated by the respective meso models 13b each contain a plurality of sarcomeres 23. The micro models 13c are models of the respective sarcomeres 23 contained in the meso model 13b. In the example of FIG. 2, each of the micro models 13c is a model having a shape of regular hexahedron and has eight node points 24.
The condition data 13d is data indicating conditions when biological simulation is performed. FIG. 3 is a diagram illustrating an example of the condition data. In an example of FIG. 3, the condition data 13d has items of "time step" and "Ca$^{2+}$ concentration". Time steps of the biological simulation are registered in the item of the "time step". Concentrations of calcium ion (Ca$^{2+}$) in each cardiomyocyte at the time steps registered in the item of the "time step" are registered in the item of "Ca$^{2+}$ concentration". That is to say, the condition data 13d as illustrated in FIG. 3 indicates the calcium ion concentration in each cardiomyocyte at each time step.
The storage unit 13 is a storage device exemplified by a semiconductor memory element such as a flash memory, a hard disk, and an optical disk. Note that the storage unit 13 is not limited to the above-mentioned kinds of storage devices and may be a random access memory (RAM), a read only memory (ROM), or the like.
The controller 14 includes an internal memory for storing programs defining various processing procedures and control data, and executes various kinds of processing by the use of them. As illustrated in FIG. 1, the controller 14 includes a plurality of macro processors 14a as second processors, a plurality of meso-micro processors 14b as first processors, and a display controller 14c.
The macro processors 14a correspond to the respective macro models 13a, and each of the macro processors 14a calculates the behavior of the corresponding macro model 13a. The meso-micro processors 14b correspond to the respective meso models 13b, and each of the meso-micro processors 14b calculates the behavior of the corresponding meso model 13b. Furthermore, each of the meso-micro processors 14b calculates the behavior of each of the micro models 13c indicating the respective sarcomeres 23 that are contained in the cardiomyocyte 21 indicated by the corresponding meso model. That is to say, in the embodiment, a plurality of meso models 13b correspond to one macro model 13a, so that a plurality of meso-micro processors 14b correspond to one macro processor 14a. Furthermore, the controller 14 includes one or a plurality of multi-core central processing unit(s) (CPU(s)) having a plurality of cores as processors. Alternatively, the controller 14 may include a plurality of single-core CPUs having one core as the processor. The cores each execute a biological simulation program described later, thereby implementing each part of the macro processors 14a, the meso-micro processors 14b, and the display controller 14c thereon.

Each of the macro processors 14a as the second processor includes a calculator 15a. Each of the meso-micro processors 14b as the first processor includes a first calculator 15b, a second calculator 15c, a first updating unit 15d, and a second updating unit 15e.

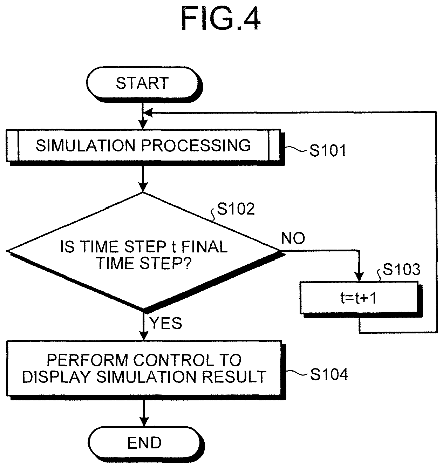

The following describes an example of processing that is executed by the controller 14 with reference to FIG. 4. FIG. 4 is a flowchart illustrating procedures of the biological simulation processing in the first embodiment. The biological simulation processing is executed when the input unit 11 inputs an instruction to execute the biological simulation processing to the controller 14, for example. As illustrated in FIG. 4, the macro processor 14a and the meso-micro processor 14b execute simulation processing (S101). The display controller 14c then determines whether a time step t in the executed simulation processing is a final time step in the biological simulation (S102). When the time step t is not the final time step (No at S102), the display controller 14c increments a value of the time step t by 1 and advances the time step by 1 (S103). The process returns to the processing at S101, and the macro processors 14a and the meso-micro processors 14b execute the simulation processing again.

When the time step t is the final time step (Yes at S102), the display controller 14c performs the following processing. The display controller 14c controls the display unit 12 to display shapes of the macro models 13a, the meso models 13b, and the micro models 13c in accordance with displacement that have been calculated in the simulation processing in the order from the first time step to the final time step (S104).

The following describes an example of the simulation processing in the embodiment. In the embodiment, multi-scale analysis between the macro models 13a and the meso models 13b is performed as follow. That is, a non-linear equation at each time step is linearized to lead to questions (the following equations (1) to (4)) that are solved repeatedly using the Newton-Raphson method.

$$\mathbf{A}_{\widetilde{\mathrm{P}},e} := \widetilde{\mathbf{P}}^{T} \mathbf{A}_{\mathrm{F},e} \widetilde{\mathbf{P}} \qquad (1)$$

$$\boldsymbol{A}_{C,e} = \boldsymbol{B}_{C,e}{}^{T} \widetilde{G}^{T} \boldsymbol{A}_{F,e} \widetilde{G} \boldsymbol{B}_{C,e} \qquad (2)$$

$$\boldsymbol{f}_{C,e} = \boldsymbol{B}_{C,e}{}^{T} \widetilde{G}^{T} \boldsymbol{f}_{F,e} \qquad (3)$$

$$\begin{bmatrix} \sum_{e} \mathbf{A}_{\widetilde{\mathrm{P}},e} & \sum_{e} \widetilde{\mathbf{P}}^{T} \mathbf{A}_{\mathrm{F},e} \widetilde{G} \mathbf{B}_{C,e} \\ \sum_{e} \mathbf{B}_{C,e}{}^{T} \widetilde{G}^{T} \mathbf{A}_{\mathrm{F},e} \widetilde{\mathbf{P}} \ \mathbf{C}_{C} + \sum_{e} \mathbf{A}_{C,e} \end{bmatrix} \begin{bmatrix} \sum_{e} \mathbf{x}_{\mathrm{F},e} \\ \mathbf{x}_{C} \end{bmatrix} = - \begin{bmatrix} \sum_{e} \widetilde{\mathbf{P}}^{T} \mathbf{f}_{\mathrm{F},e} \\ \mathbf{g}_{C} + \sum_{e} \mathbf{f}_{C,e} \end{bmatrix} \qquad (4)$$

$A_{F,e}$ is a stiffness matrix (meso element stiffness matrix) corresponding to an element e in the meso model 13b. $A_{C,e}$ is a stiffness matrix (macro element stiffness matrix) corresponding to an element e in the macro model 13a. $f_{F,e}$ is a meso equivalent nodal force corresponding to $A_{F,e}$. $f_{C,e}$ is a macro equivalent nodal force corresponding to $A_{C,e}$. $x_{F,e}$ is a solution (meso solution) of the element e in the meso model 13b, for example, a displacement of the element e in the meso model 13b. $x_{C}$ is a solution (macro solution) of the macro model 13a, for example, a displacement of the macro model 13a.

$\widetilde{G}$ is an operator for obtaining a stress on a meso coordinate system from the strain of the macro model 13a. $B_{C,e}$ is an operator for obtaining a strain from the displacement of the element e in the macro model 13a.

$\widetilde{P}$ is an operator indicating embedding into meso period displacement. $C_{C}$ is a stiffness matrix (macro stiffness matrix) corresponding to the macro model 13a. $g_{C}$ is a macro equivalent nodal force corresponding to $C_{C}$.

When the equations are solved repeatedly using the Newton-Raphson method, a vector $\chi_{C,e}$ called a characteristic displacement mode is calculated in accordance with the following equation (5).

$$\boldsymbol{A}_{\widetilde{P},e} \chi_{C,e} = \widetilde{\boldsymbol{P}}^{T} \boldsymbol{A}_{F,e} \widetilde{G} \qquad (5)$$

A Schur complement $S_{C,e}$ is calculated in accordance with the following equation (6) using nature that the meso solutions $x_{F,e}$ give no influence on one another directly.

$$S_{C,e} = A_{C,e} - B_{C,e}{}^{T} \widetilde{G}^{T} \boldsymbol{A}_{F,e} \widetilde{P} \chi_{C,e} \boldsymbol{B}_{C,e} \qquad (6)$$

Subsequently, block lower triangle matrix upper triangle matrix (LU) factorization is performed using $S_{C,e}$, in the manner as indicated by the following equation (7). The block LU factorization is a method of factorizing the square matrix into a product of a lower triangular block matrix and an upper triangular block matrix in the idea that a square matrix is a group of sub-matrices (blocks). The lower triangular block matrix indicates a matrix that all the upper-right blocks relative to a diagonal block are null matrices, and the upper triangular block matrix indicates a matrix that all the lower-left blocks relative to the diagonal block are null matrices. Noted that the LU factorization can be also considered as block LU factorization when the size of all the block matrices is set to $1 \times 1$.

$$\widetilde{M} = \begin{bmatrix} A_{\widetilde{P},e} & \widetilde{P}^T A_{F,e} \widetilde{G} B_{C,e} \\ B_{C,e}^T \widetilde{G}^T A_{F,e} \widetilde{P} & A_{C,e} \end{bmatrix} = \begin{bmatrix} A_{\widetilde{P},e} & 0 \\ B_{C,e}^T \widetilde{G}^T A_{F,e} \widetilde{P} & S_{C,e} \end{bmatrix} \begin{bmatrix} I & \chi_{C,e} B_{C,e} \\ 0 & I \end{bmatrix} \quad (7)$$

When the equation (4) is deformed using the equation (7), the meso solutions $x_{F,e}$ can be calculated, the macro solution $x_C$ can be calculated, and the meso solutions $x_{F,e}$ can be updated using the following equations (8) to (11). Specifically, the macro solution $x_C$ can be calculated based on the calculation results of the meso solutions $x_{F,e}$, and the calculation result of the macro solution $x_C$ can be reflected to the meso solutions $x_{F,e}$. When the meso solutions $x_{F,e}$ are calculated using the equation (8), the meso solution $x_{F,e}$ can be calculated independently for each element e of the meso model. Thus, when a core is assigned to each of the meso-micro processors 14b and the cores calculate the meso solutions $x_{F,e}$ in parallel as in the embodiment, the meso solutions $x_{F,e}$ can be calculated at high speed because the meso solutions $x_{F,e}$ are calculated in parallel. The embodiment can therefore provide highly efficient calculation of the meso solutions $x_{F,e}$.

$$A_{\widetilde{P},e} x_{F,e} = -\widetilde{P}^T f_{F,e} \qquad (8)$$

$$b_{C,e} := f_{C,e} + B_{C,e}^T \widetilde{G}^T A_{F,e} \widetilde{P} x_{F,e} \qquad (9)$$

$$\left( C_C + \sum_e S_{C,e} \right) x_C = -g_C - \sum_e b_{C,e} \qquad (10)$$

$$x_{F,e} := x_{F,e} - \chi_{C,e} B_{C,e} x_C \qquad (11)$$

In the embodiment, also in the multi-scale analysis between the meso models 13b and the micro models 13c, a non-linear equation at each step is linearized to lead to questions (the following equations (12) to (15)) that are solved repeatedly using the Newton-Raphson method.

$$\boldsymbol{A}_{P,e} := \boldsymbol{P}^T \boldsymbol{A}_{M,e} \boldsymbol{P} \qquad (12)$$

$$\boldsymbol{A}_{F,e} = \boldsymbol{B}_{F,e}^T G^T \boldsymbol{A}_{M,e} G \boldsymbol{B}_{F,e} \qquad (13)$$

$$\boldsymbol{f}_{F,e} = \boldsymbol{B}_{F,e}^T G^T \boldsymbol{f}_{M,e} \qquad (14)$$

$$\begin{bmatrix} \sum_e \boldsymbol{A}_{P,e} & \sum_e \boldsymbol{P}^T \boldsymbol{A}_{M,e} G \boldsymbol{B}_{F,e} \\ \sum_e \boldsymbol{B}_{F,e}^T G^T \boldsymbol{A}_{M,e} \boldsymbol{P} \, \boldsymbol{C}_F + \sum_e \boldsymbol{A}_{F,e} \end{bmatrix} \begin{bmatrix} \sum_e \boldsymbol{x}_{M,e} \\ \boldsymbol{x}_F \end{bmatrix} = - \begin{bmatrix} \sum_e \boldsymbol{P}^T \boldsymbol{f}_{M,e} \\ \boldsymbol{g}_F + \sum_e \boldsymbol{f}_{F,e} \end{bmatrix} \qquad (15)$$

$A_{M,e}$ is a stiffness matrix (micro element stiffness matrix) corresponding to an element e of the micro model 13c. $f_{M,e}$ is a micro equivalent nodal force. $x_{M,e}$ is a solution (micro solution) of the element e in the micro model 13c, for example, a displacement of the element e in the micro model 13c. G is an operator for obtaining a stress on a micro coordinate system from a strain of the meso model 13b. $B_{F,e}$ is an operator for obtaining a strain from the displacement of the element e in the meso model 13b. P is an operator indicating embedding into meso period displacement. $C_F$ is a stiffness matrix (meso stiffness matrix) of the meso model 13b. $g_F$ is a meso equivalent nodal force corresponding to $C_F$.

When the equations are solved repeatedly using the Newton-Raphson method, a vector $\chi_{F,e}$ called characteristic displacement modes are calculated in accordance with the following equation (16).

$$\boldsymbol{A}_{P,e}\chi_{F,e} = \boldsymbol{P}^T\boldsymbol{A}_{M,e}G \qquad (16)$$

A Schur complements $S_{F,e}$ is calculated in accordance with the following equation (17) using nature that the micro solutions $x_{M,e}$ give no influence on one another directly.

$$S_{F,e} = A_{F,e} - B_{F,e}^T G^T \boldsymbol{A}_{M,e} P \chi_{F,e} \boldsymbol{B}_{F,e} \qquad (17)$$

Subsequently, LU factorization is performed using $S_{F,e}$ in the manner as indicated by the following equation (18).

$$M = \begin{bmatrix} A_{P,e} & P^T A_{M,e} G B_{F,e} \\ B_{F,e}^T G^T A_{M,e} P & A_{F,e} \end{bmatrix} = \begin{bmatrix} A_{P,e} & 0 \\ B_{F,e}^T G^T A_{M,e} P & S_{F,e} \end{bmatrix}\begin{bmatrix} I & \chi_{F,e} B_{F,e} \\ 0 & I \end{bmatrix} \qquad (18)$$

When the equation (15) is deformed using the equation (18), the micro solutions $x_{M,e}$ can be calculated, the meso solution $x_{F,e}$ can be calculated, and the micro solutions $x_{M,e}$ can be updated using the following equations (19) to (22). Specifically, the meso solution $x_{F,e}$ is calculated based on the calculation results of the micro solutions $x_{M,e}$, and the calculation result of the meso solution $x_{F,e}$ can be reflected to the micro solutions $x_{M,e}$.

$$\boldsymbol{A}_{P,e}x_{M,e} = -\boldsymbol{P}^T f_{M,e} \qquad (19)$$

$$b_{F,e} := f_{F,e} + B_{F,e}^T G^T A_{M,e} P x_{M,e} \qquad (20)$$

$$\left(C_F + \sum_e S_{F,e}\right)x_F = -g_F - \sum_e b_{F,e} \qquad (21)$$

$$x_{M,e} := x_{M,e} - \chi_{F,e} B_{F,e} x_F \qquad (22)$$

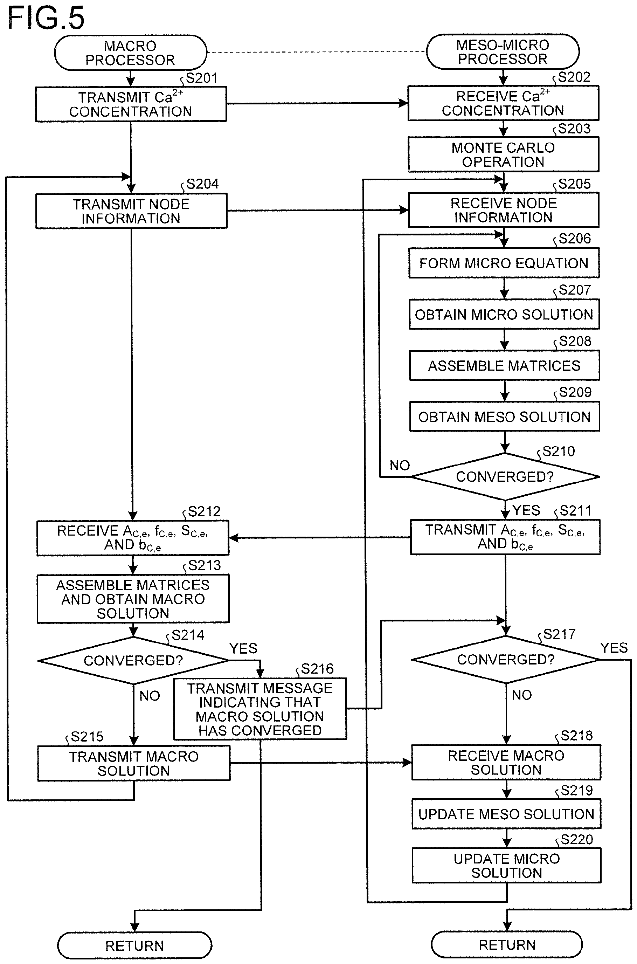

Described is an example of the above-mentioned simulation processing that is executed by the macro processors 14a and the meso-micro processors 14b. FIG. 5 is a flowchart illustrating procedures of the simulation processing in the first embodiment. In the simulation processing in the embodiment, information transfer occurs between the macro processors 14a and the respective meso-micro processors 14b. Note that the example of FIG. 5 illustrates processing that is executed by one macro processor 14a and processing that is executed by one meso-micro processor 14b for the convenience of description.

As illustrated in FIG. 5, the calculator 15a of the macro processor 14a acquires the calcium ion concentration for each of the cardiomyocytes at the current time step t from the condition data 13d and transmits the acquired calcium ion concentrations to the respective meso-micro processors 14b corresponding to the respective cardiomyocytes (S201).

The first calculator 15b of each of the meso-micro processors 14b receives the calcium ion concentration transmitted from the calculator 15a (S202). The first calculator 15b then calculates the contraction force and the like for each of the micro models 13c using the Monte Carlo method (S203). The calculator 15a transmits node information including the pressures of respective nodes to each of the meso-micro processors 14b (S204).

Subsequently, the first calculator 15b receives the node information transmitted from the calculator 15a (S205). The first calculator 15b then calculates the vectors $\chi_{F,e}$ using the equation (16) (S206). The first calculator 15b calculates the Schur complement $S_{F,e}$ in accordance with the equation (17) at S206. Thereafter, the first calculator 15b performs the LU factorization using $S_{F,e}$ to obtain the equation (18) at S206. The first calculator 15b deforms the equation (15) using the equation (18) to obtain the equations (19) to (22) at S206. The first calculator 15b then calculates $b_{F,e}$ in accordance with the equation (20) at S206.

Subsequently, the first calculator 15b calculates the micro solutions $x_{M,e}$ in accordance with the equation (19) (S207).

The second calculator 15c of the meso-micro processor 14b calculates the vector $\chi_{C,e}$ using the equation (5) (S208). The second calculator 15c calculates the Schur complement $S_{C,e}$ in accordance with the equation (6) at S208. Thereafter, the second calculator 15c performs the LU factorization using $S_{C,e}$ to obtain the equation (7) at S208. The second calculator 15c then deforms the equation (4) using the equation (7) to obtain the equation (8), the equation (9), and the equation (11) at S208. The second calculator 15c calculates $b_{C,e}$ in accordance with the equation (9) at S208.

Subsequently, the second calculator 15c calculates the meso solutions $x_{F,e}$ of the respective elements of the meso model 13b in accordance with the equation (8), and calculates the meso solution $x_F$ of the meso model 13b from the meso solutions $x_{F,e}$ of the elements of the meso model 13b in accordance with the equation (21) (S209). The second calculator 15c then determines whether the meso solution $x_F$ has converged (S210). When the meso solution $x_F$ has not converged (No at S210), the process returns to S206. When the meso solution $x_F$ has converged (Yes at S210), the second calculator 15c transmits $A_{C,e}$ obtained as the result of calculation of the equation (2), $f_{C,e}$ obtained as the result of calculation of the equation (3), the Schur complement $S_{C,e}$, and $b_{C,e}$ to the macro processor 14a (S211).

Upon receiving $A_{C,e}$, $f_{C,e}$, $S_{C,e}$, and $b_{C,e}$ from all the meso-micro processors 14b corresponding to the macro processor 14a (S212), the calculator 15a of the macro processor 14a deforms the equation (4) using the equation (7) to obtain the equation (10) (S213). The calculator 15a calculates the macro solution $x_C$ in accordance with the equation (10) at S213. The calculator 15a then determines whether the macro solution $x_C$ has converged (S214). When the macro solution $x_C$ has not converged (No at S214), the calculator 15a transmits the macro solution $x_C$ to each of the meso-micro processors 14b (S215). The process returns to S204, and the calculator 15a updates node information including the pressures and the like of the respective nodes and transmits the updated node information to each of the meso-micro processors 14b. When the macro solution $x_C$ has converged (Yes at S214), the calculator 15a transmits a message indicating that the macro solution $x_C$ has converged to each of the meso-micro processors 14b (S216), and stores a processing result in the internal memory. The process then returns.

When the processing at S211 is executed, the first updating unit 15d of each of the meso-micro processors 14b determines whether it has received the message indicating that the macro solution $x_C$ has converged so as to determine whether the macro solution $x_C$ has converged (S217). Upon receiving the message indicating that the macro solution $x_C$ has converged, the first updating unit 15d determines that the macro solution $x_C$ has converged. Upon receiving no message indicating that the macro solution $x_C$ has converged, the first updating unit 15d determines that the macro solution $x_C$ has not converged.

When the macro solution $x_C$ has not converged (No at S217), the first updating unit 15d receives the macro solution $x_C$ from the calculator 15a (S218), and substitutes the received macro solution $x_C$ into the equation (11) to update the meso solutions $x_{F,e}$ (S219).

Subsequently, the second updating unit 15e of each of the meso-micro processors 14b substitutes the meso solution $x_F$ into the equation (22) to update the micro solutions $x_{M,e}$ (S220), and stores a processing result in the internal memory. The process then returns.

As described above, the biological simulation device 10 according to the embodiment calculates the behavior of the sarcomeres, which are indicated by the micro models 13c, contained in each of the cardiomyocytes. The biological simulation device 10 calculates the behavior of the cardiomyocytes indicated by the meso models 13b based on the calculated behaviors of the sarcomeres. Subsequently, the biological simulation device 10 calculates the behavior of the heart indicated by the macro models 13a based on the calculated behaviors of the cardiomyocytes. Thereafter, the biological simulation device 10 updates the behaviors of the cardiomyocytes indicated by the meso models 13b based on the calculated behavior of the heart. The biological simulation device 10 then updates the behaviors of the sarcomeres indicated by the micro models 13c based on the updated behaviors of the cardiomyocytes. Thus, the biological simulation device 10 performs simulation of the behavior using models of three levels including the macro models 13a indicating the entire heart, the meso models 13b indicating the cardiomyocytes, and the micro models 13c indicating the sarcomeres. Thus, the biological simulation device 10 can perform accurate simulation, that is, multi-scale analysis of three levels. This enables the biological simulation device 10 to provide an accurate simulation result.

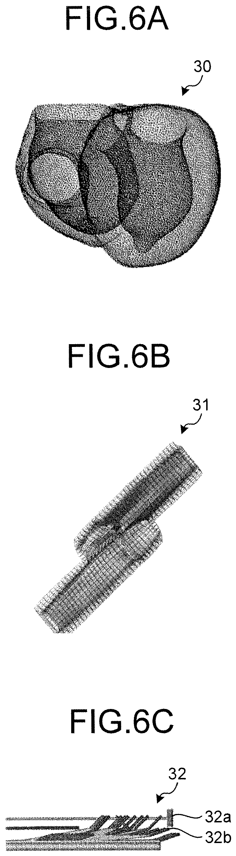

FIG. 6A to FIG. 6C illustrate examples of a simulation result of each model at a certain time step that is displayed on the display unit. FIG. 6A illustrates the shape of a heart 30 indicated by the macro models 13a at a certain time step that is displayed on the display unit 12. FIG. 6B illustrates the shape of a cardiomyocyte 31 indicated by the meso model 13b at a certain time step that is displayed on the display unit 12. FIG. 6C illustrates the shape of a sarcomere 32 indicated by the micro model 13c at a certain time step that is displayed on the display unit 12. As illustrated in FIG. 6C, with the biological simulation device 10 according to the embodiment, a structure of a portion is visible on which a cross bridge of actin filaments 32a and myosin heads 32b is finely formed.

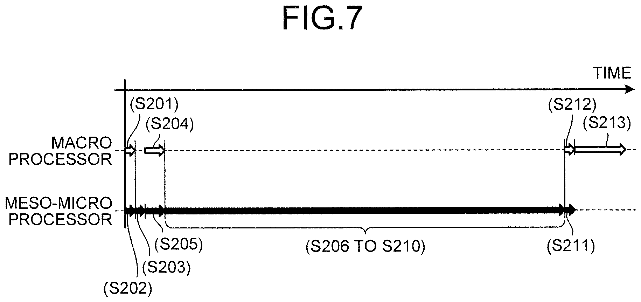

The following describes an example of execution timings of pieces of processing by the macro processor 14a and the meso-micro processor 14b in the first embodiment. FIG. 7 is a time chart illustrating an example of the execution timings of the pieces of processing by the macro processor and the meso-micro processor in the first embodiment. The example of FIG. 7 illustrates times and execution timings of the processing at the steps S that are executed by the macro processor 14a and times and execution timings of the processing at the steps S that are executed by the meso-micro processor 14b. The time chart illustrated in FIG. 7 indicates that the macro processor 14a do not execute processing and is in a standby state while the meso-micro processor 14b execute the processing at S206 to S210.

[b] Second Embodiment

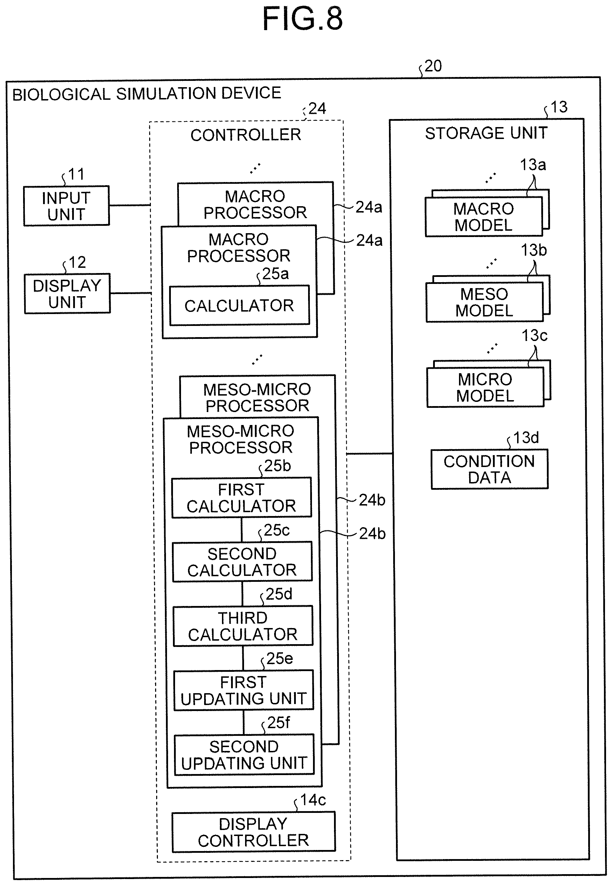

As a second embodiment, the following describes a biological simulation device that can reduce the time during which the macro processor is in the standby state. The same reference numerals as those in the first embodiment denote the same components, and description thereof is omitted in some cases. FIG. 8 is a diagram illustrating an example of a functional configuration of the biological simulation device according to the second embodiment. This biological simulation device 20 includes an input unit 11, a display unit 12, a storage unit 13, and a controller 24.

The input unit 11, the display unit 12, and the storage unit 13 have the same configurations as those in the first embodiment, so that description thereof is omitted.

The controller 24 includes an internal memory for storing programs defining various processing procedures and control data, and executes various pieces of processing by the use of them. As illustrated in FIG. 8, the controller 24 includes a plurality of macro processors 24a, a plurality of meso-micro processors 24b, and a display controller 14c.

The macro processors 24a correspond to the macro models 13a, respectively, and each of the macro processors 24a calculates the behavior of the corresponding macro model 13a. The meso-micro processors 24b correspond to the meso models 13b, respectively, and each of the meso-micro processors 24b calculates the behavior of the corresponding meso model 13b. Furthermore, each of the meso-micro processors 24b calculates the behaviors of the respective micro models 13c indicating the respective sarcomeres 23 that are contained in the cardiomyocyte 21 indicated by the corresponding meso model. That is to say, in the embodiment, a plurality of meso models 13b correspond to one macro model 13a, so that a plurality of meso-micro processors 24b correspond to one macro processor 24a. Furthermore, the controller 24 includes a plurality of multi-core CPUs. The cores each execute a biological simulation program described later, thereby functioning each part of the macro processors 24a, the meso-micro processors 24b, and the display controller 14c thereon.

Each of the macro processors 24a includes a calculator 25a. Each of the meso-micro processors 24b includes a first calculator 25b, a second calculator 25c, a third calculator 25d, a first updating unit 25e, and a second updating unit 25f.

The biological simulation device 20 according to the second embodiment executes the biological simulation processing illustrated in the flowchart in the above-mentioned example of FIG. 4 as in the first embodiment, so that description of the biological simulation processing is omitted. The simulation processing in the second embodiment is different from the simulation processing in the first embodiment. The following describes an example of the simulation processing in the second embodiment. There are different points between the simulation processing in the second embodiment and the simulation processing in the first embodiment, which will be described below. In the second embodiment, the following equation (23) is used instead of the equation (7) in the first embodiment. The equation (23) is an equation as a result of performing approximate LU factorization.

$$\widetilde{N} = \begin{bmatrix} A_{\widetilde{P},e} & 0 \\ 0 & S_{C,e} \end{bmatrix} \begin{bmatrix} I & \chi_{C,e}B_{C,e} \\ 0 & I \end{bmatrix} = \begin{bmatrix} A_{\widetilde{P},e} & \widetilde{P}^{T} A_{F,e}\widetilde{G}B_{C,e} \\ 0 & A_{C,e} \end{bmatrix} \qquad (23)$$

Furthermore, while $b_{C,e}$ is calculated using the equation (9) at S208 in the first embodiment, $b_{C,e}$ is not calculated in the second embodiment. While the macro solution $x_C$ is calculated using the equation (10) at S213 in the first embodiment, the macro solution $x_C$ is calculated using the following equation (24) instead of the equation (10) in the second embodiment.

$$\left( C_C + \sum_e S_{C,e} \right) x_C = -g_C - \sum_e f_{C,e} \qquad (24)$$

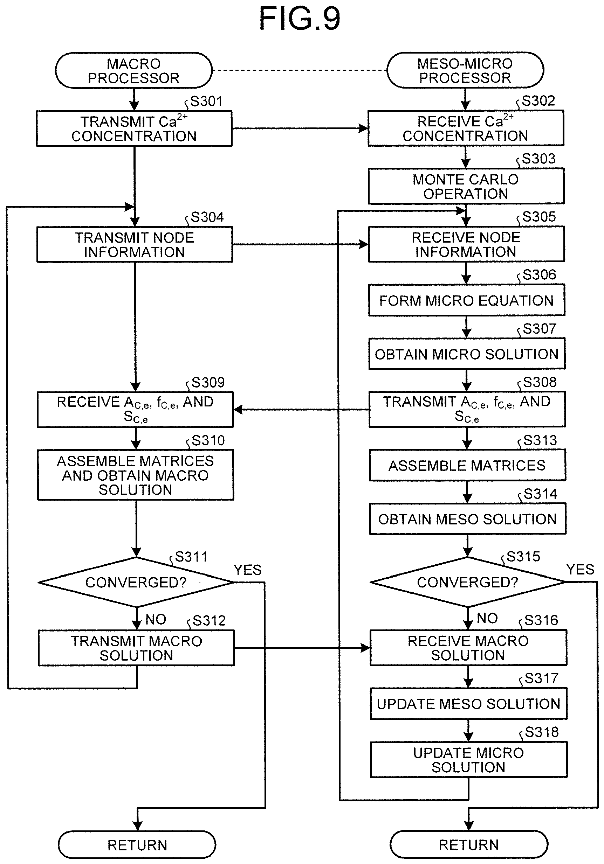

The following describes an example of the above-mentioned simulation processing that is executed by the macro processors 24a as second processors and the meso-micro processors 24b as first processors. FIG. 9 is a flowchart illustrating procedures of the simulation processing in the second embodiment. Also in the simulation processing in the second embodiment, information transfer occurs between the macro processors 24a and the respective meso-micro processors 24b. Note that the example of FIG. 9 illustrates processing that is executed by one macro processor 24a and processing that is executed by one meso-micro processor 24b for the convenience of description.

As illustrated in FIG. 9, the calculator 25a of the macro processor 24a acquires the calcium ion concentrations of the respective cardiomyocytes at the current time step t from the condition data 13d and transmits the acquired calcium ion concentrations to the respective meso-micro processors 24b corresponding to the respective cardiomyocytes (S301).

The first calculator 25b of each of the meso-micro processors 24b receives the calcium ion concentration transmitted from the calculator 25a (S302). The first calculator 25b calculates the contraction force and the like for each of the micro models 13c using the Monte Carlo method (S303). The calculator 25a transmits node information including the pressures of respective node to each of the meso-micro processors 24b (S304).

Subsequently, the first calculator 25b receives the node information transmitted from the calculator 25a (S305). The first calculator 25b then calculates the vectors $\chi_{F,e}$ using the equation (16) (S306). The first calculator 25b calculates the Schur complement $S_{F,e}$ in accordance with the equation (17) at S306. Thereafter, the first calculator 25b performs the LU factorization using $S_{F,e}$ to obtain the equation (18) at S306. The first calculator 25b deforms the equation (15) using the equation (18) to obtain the equations (19) to (22) at S306. The first calculator 25b then calculates $b_{F,e}$ in accordance with the equation (20) at S306.

Subsequently, the first calculator 25b calculates the micro solutions $x_{M,e}$ in accordance with the equation (19) (S307). The second calculator 25c of each of the meso-micro processors 24b calculates the vector $\chi_{C,e}$ using the equation (5) at S307. The second calculator 25c calculates the Schur complement $S_{C,e}$ in accordance with the equation (6) at S307. Thereafter, The second calculator 25c performs the LU factorization using $S_{C,e}$ to obtain the equation (23) at S307. The third calculator 25d of each of the meso-micro processors 24b calculates $f_{C,e}$ using the equation (3).

Subsequently, the third calculator 25d transmits $A_{C,e}$ obtained as a result of the calculation of the equation (2), $f_{C,e}$ as a result of the calculation of the equation (3), and the Schur complement $S_{C,e}$ to the macro processor 24a (S308).

Upon receiving $A_{C,e}$, $f_{C,e}$ and $S_{C,e}$ from all the meso-micro processors 24b corresponding to the macro processor 24a (S309), the calculator 25a of the macro processor 24a deforms the equation (4) using the equation (23) to obtain the equation (24) (S310). The calculator 25a calculates the macro solution $x_C$ in accordance with the equation (24) at S310. The calculator 25a then determines whether the macro solution $x_C$ has converged (S311). When the macro solution $x_C$ has not converged (No at S311), the calculator 25a transmits the macro solution $x_C$ to each of the meso-micro processors 24b (S312). The process returns to S304, and the calculator 25a updates node information including the pressures and the like of respective nodes and transmits the updated node information to each of the meso-micro processors 24b. When the macro solution $x_C$ has converged (Yes at S311), the calculator 25a stores a processing result in the internal memory. The process then returns.

When the processing at 308 is executed, the second calculator 25c deforms the equation (4) using the equation (23) to obtain the equation (8) and the equation (11) (S313).

The second calculator 25c calculates the meso solutions $x_{F,e}$ of the respective elements of the meso model 13b in accordance with the equation (8), and calculates the meso solution $x_F$ of the meso model 13b from the meso solutions $x_{F,e}$ of the respective elements of the meso model 13b in accordance with the equation (21) (S314). The second calculator 25c then determines whether the meso solution $x_F$ has converged (S315). When the meso solution $x_F$ has not converged (No at S315), the first updating unit 25e of each of the meso-micro processors 24b receives the macro solution $x_C$ from the calculator 25a (S316), and substitutes the received macro solution $x_C$ into the equation (11) to update the meso solutions $x_{F,e}$ (S317).

Subsequently, the second updating unit 25f of each of the meso-micro processors 24b substitutes the meso solution $x_F$ into the equation (22) to update the micro solutions $x_{M,e}$ (S318), and stores a processing result in the internal memory. The process then returns.

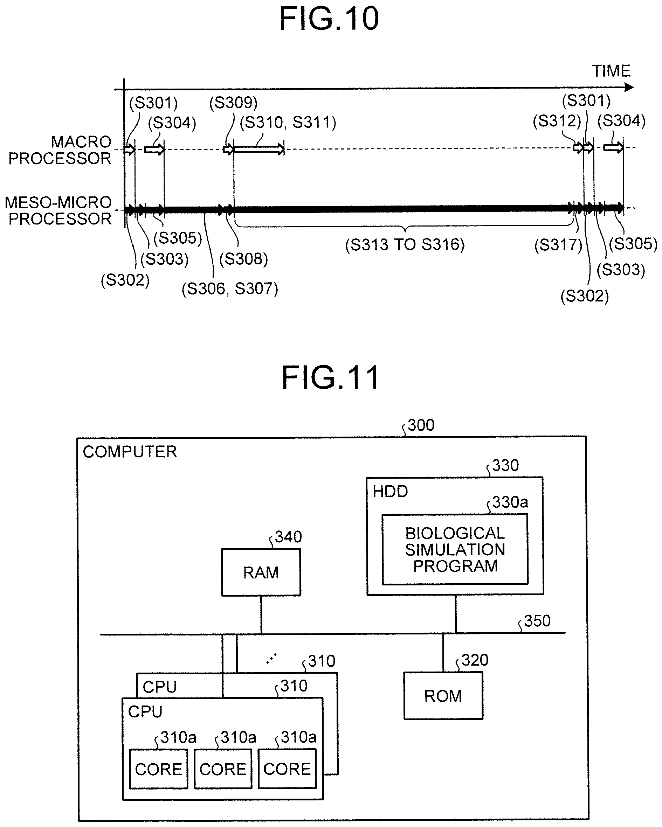

The following describes an example of execution timings of pieces of processing by the macro processor 24a and the meso-micro processor 24b in the second embodiment. FIG. 10 is a time chart illustrating an example of the execution timings of pieces of processing by the macro processor and the meso-micro processor in the second embodiment. The example of FIG. 10 illustrates times and execution timings of the processing at the steps S that are executed by the macro processor 24a and times and execution timings of the processing at the steps S that are executed by the meso-micro processor 24b. In the embodiment, the equation (4) is deformed by the use of the following equation (23) instead of the equation (7) in the first embodiment to obtain an equation (24). In the embodiment, the macro solution $x_C$ is calculated using the equation (24). For this reason, as illustrated in FIG. 10, the macro processor 24a can execute the processing at S310 and S311 while the meso-micro processor 24b executes the processing at S206 to S210. That is to say, in the embodiment, it is possible to execute the processing of calculating the meso solution $x_F$ by the meso-micro processor 24b and the processing of calculating the macro solution $x_C$ by the macro processor 24a in parallel. As a result, the biological simulation device 20 according to the second embodiment can prevent increase in the calculation time. Furthermore, the biological simulation device 20 according to the embodiment can prevent lowering of the utilization rate of the cores of the CPU.

As described above, the biological simulation device 20 according to the embodiment calculates the behavior of the sarcomeres, which are indicated by the micro models 13c, contained in each of the cardiomyocytes. The biological simulation device 20 calculates a force that is applied to the heart indicated by the macro model 13a. The biological simulation device 20 then calculates the behavior of the heart based on the calculated force. Subsequently, the biological simulation device 20 calculates the behavior of the cardiomyocytes indicated by the meso models 13b based on the calculated behaviors of the sarcomeres. Thereafter, the biological simulation device 20 updates the behaviors of the cardiomyocytes indicated by the meso models 13b based on the calculated behavior of the heart. The biological simulation device 20 then updates the behaviors of the sarcomeres indicated by the micro models 13c based on the updated behaviors of the cardiomyocytes. Thus, the biological simulation device 20 performs simulation of the behavior using models of three levels including the macro models 13a indicating the entire heart, the meso models 13b indicating the cardiomyocytes, and the micro models 13c indicating the sarcomeres. Thus, the biological simulation device 20 can perform accurate simulation, that is, multi-scale analysis of three levels. This enables the biological simulation device 20 to provide an accurate simulation result.

Furthermore, as described above, with the biological simulation device 20 in the second embodiment, the macro processor 24a can execute the processing at S310 and S311 while the meso-micro processor 24b executes the processing at S206 to S210. That is to say, in the second embodiment, it is possible to execute the processing of calculating the meso solution $x_F$ by the meso-micro processor 24b and the processing of calculating the macro solution $x_C$ by the macro processor 24a in parallel. This enables the biological simulation device 20 according to the second embodiment to prevent increase in the calculation time. Furthermore, the biological simulation device 20 according to the second embodiment can prevent lowering of the utilization rate of the cores of the CPU.

Although the embodiments relating to the disclosed device have been described hereinbefore, the device of the invention may be executed in various different modes other than the above-mentioned embodiments. The following describes other embodiments that are encompassed in the invention.

For example, although one meso-micro processor 14b or 24b is executed on one core and one macro processor 14a or 24a is executed on one core in the above-mentioned first embodiment and second embodiment, the disclosed device is not limited thereto. For example, a plurality of meso-micro processors 14b or 24b may be executed on one core. A plurality of macro processors 14a or 24a may be executed on one core. Alternatively, the meso-micro processor 14b or 24b and the macro processor 14a or 24a may be executed on one core.

The entire processing or a part of the processing described in the above-mentioned embodiments that is performed automatically may be performed manually. The entire processing or a part of the processing described in the above-mentioned embodiments that is performed manually can be performed automatically with a known method.

The processing at the steps in the processes described in the above-mentioned embodiments can be subdivided into small pieces or be compiled as appropriate depending on various loads or usage conditions. Any of the steps can be omitted.

Furthermore, the order of the pieces of processing at the steps in the processes described in the above-mentioned embodiments can be changed depending on various loads or usage conditions.

The components of the devices in the drawings are illustrated conceptually in function and are not necessarily configured as illustrated in the drawings physically. That is to say, the specific state of separation and integration of the devices are not limited to those illustrated in the drawings. The whole or a part thereof can be separated or integrated functionally or physically based on any desired unit depending on various loads or usage conditions.

Biological Simulation Program

Various pieces of processing in the biological simulation device 10/20 described in the above-mentioned embodiments can be implemented through execution of a previously prepared program on a computer system such as a personal computer and a workstation. The following describes an example of a computer that executes a computer program having the same functions as those in the biological simulation device as described in the above-mentioned embodiments with reference to FIG. 11. FIG. 11 is a diagram illustrating the computer that executes a biological simulation program.

As illustrated in FIG. 11, a computer 300 includes a plurality of multi-core CPUs 310, a ROM 320, a hard disk drive (HDD) 330, and a RAM 340. These components 310 to 340 are connected to one another via a bus 350. The CPU 310 includes a plurality of cores 310a.

The ROM 320 stores therein a basic program such as an OS. The HDD 330 previously stores therein a biological simulation program 330a exhibiting the same functions as those of the calculator 15a, the first calculator 15b, the second calculator 15c, the first updating unit 15d, and the second updating unit 15e in the above-mentioned first embodiment. Note that the biological simulation program 330a may be separated as appropriate. The biological simulation program 330a may be a computer program exhibiting the same functions as those of the calculator 25a, the first calculator 25b, the second calculator 25c, the third calculator 25d, the first updating unit 25e, and the second updating unit 25f in the above-mentioned second embodiment.

The CPUs 310 load the biological simulation program 330a from the HDD 330 and execute it.

The above-mentioned biological simulation program 330a is not necessarily stored in the HDD 330 initially.

For example, the computer 300 may load the biological simulation program 330a from the biological simulation program 330a stored in a "mobile physical medium" such as a flexible disk (FD), a compact disk read only memory (CD-ROM), a digital versatile disk (DVD), a magnetooptical disk, and an IC card that is inserted into the computer 300, and execute it.

Furthermore, the computer 300 may load the biological simulation program 330a from the biological simulation program 330a stored in "another computer (or server)" that is connected to the computer 300 through a public line, the Internet, a LAN, a wide area network (WAN), or the like, and execute it.

An accurate simulation result can be obtained.

## Claims

1. A biological simulation program which, when executed by a computer having a first processor (14b, 24b) and a second processor (14a, 24a), causes the computer to perform a biological simulation method comprising:

   calculating (S207, S307) a behavior of a micro model (13c) that has node points (24) and that is a model of a material contained in a cell of an organ of a biological body, the organ being modelled by a macro model (13a) that has node points, the calculating calculates displacements ($x_{M,e}$) of the node points (24) based on a simulation result of the micro model (13c) at each time step;
   calculating a force that is applied to the node points of the macro model (13a);
   calculating (S213, S310) a behavior of the macro model (13a) based on the calculated force that is applied to the node points of the macro model (13a), the calculating calculates displacements ($x_c$) of the node points based on a simulation result of the macro model (13a) at each time step;
   calculating (S209, S314) a behavior of a meso model (13b) that has node points (22) and that is a model of the cell, based on the calculated behavior of the micro model (13c) used in a biological simulation of the material, the calculating calculates displacements ($x_{F,e}$) of the node points (22) based on a simulation result of the meso model (13b) at each time step;
   updating (S219, S317) the behavior of the meso model (13b) of the cell, based on the calculated behavior of the macro model (13a) used in a biological simulation of the organ, in accordance with displacements ($x_c$, $x_{F,e}$) of the node points of the macro model (13a) and the meso model (13b); and
   updating (S220, S318) the behavior of the micro model (13c) of the material, based on the updated behavior of the meso model (13b) used in a biological simulation of the cell, in accordance with displacements ($x_c$, $x_{F,e}$) of the node points of the meso model (13b) and the macro model (13a), wherein
   the biological simulation program causes:

      the first processor (14b, 24b) to calculate the behavior of the macro model (13a), and
      the second processor (14a, 24a) to calculate the behavior of the micro model (13c), calculate the force that is applied to node points of the macro model (13a), calculate the behavior of the meso model (13b), update the behavior of the meso model (13b), and update the behavior of the micro model (13c).

2. A biological simulation device, comprising:

   a first processor (14b, 24b); and
   a second processor (14a, 24a), wherein
   the first processor (14b, 24b) and the second processor (14a, 24a) execute a process comprising:

      calculating (S207, S307), by the second processor (14a, 24a), a behavior of a micro model (13c) that has node points (24) and that is a model of a material contained in a cell of an organ of a biological body, the organ being modelled by a macro model (13a) that has node points, the calculating calculates displacements ($x_{M,e}$) of the node points based on a simulation result of the micro model (13c) at each time step;
      calculating, by the second processor (14a, 24a), a force that is applied to the node points of the macro model (13a);
      calculating (S213, S310), by the first processor (14b, 24b), a behavior of the macro model (13a) based on the calculated force that is applied to the node points of the macro model (13a), the calculating calculates displacements ($x_c$) of the node points based on a simulation result of the macro model (13a) at each time step; calculating (S209, S314), by the second processor (14a, 24a), a behavior of a meso model (13b) that has node points (22) and that is a model of the cell, based on the calculated behavior of the micro model (13c) used in a biological simulation of the material, the calculating calculates displacements ($x_{F,e}$) of the node points of the macro model (13a);

updating (S219, S317), by the second processor (14a, 24a), the behavior of the meso model (13b) of the cell, based on the calculated behavior of the macro model (13a) used in a biological simulation of the organ, in accordance with displacements ($x_c$, $x_{F,e}$) of the node points of the macro model (13a) and the meso model (13b); and

updating (S220, S318), by the second processor (14a, 24a), the behavior of the micro model (13c) of the material, based on the updated behavior of the meso model (13b) used in a biological simulation of the cell, in accordance with displacements ($x_c$, $x_{F,e}$) of the node points of the meso model (13b) and the macro model (13c),

wherein any of the calculating of the behavior of the micro model (13c), the calculating of the behavior of the macro model (13a), the calculating of the behavior of the meso model (13b), the updating of the behavior of the meso model (13b), and the updating of the behavior of the micro model (13c) by the first processor (14b, 24b) and the calculating of the force that is applied to the node points of the macro model (13a) by the second processor (14a, 24a) are executed in parallel.

**Patentansprüche**

1. Biologisches Simulationsprogramm, welches bei Ausführung durch einen Computer, der einen ersten Prozessor (14b, 24b) und einen zweiten Prozessor (14a, 24a) hat, bewirkt, dass der Computer ein biologisches Simulationsverfahren ausführt, umfassend:

    Berechnen (S207), S307) eines Verhaltens eines Mikromodells (13c), das Knotenpunkte (24) hat und das ein Modell eines Materials ist, welches in einer Zelle eines Organs eines biologischen Körpers enthalten ist, wobei das Organ durch ein Makromodell (13a) modelliert wird, das Knotenpunkte hat, wobei die Berechnung Verschiebungen ($x_{M,e}$) der Knotenpunkte (24) auf der Basis eines Simulationsergebnisses des Mikromodells (13c) bei jedem Zeitschritt berechnet;
    Berechnen einer Kraft, die auf die Knotenpunkte des Makromodells (13a) ausgeübt wird;
    Berechnen (S213, S310) eines Verhaltens des Makromodells (13a) auf der Basis der berechneten Kraft, die auf die Knotenpunkte des Makromodells (13a) ausgeübt wird, wobei das Berechnen Verschiebungen ($x_c$) der Knotenpunkte auf der Basis eines Simulationsergebnisses des Makromodells (13a) zu jedem Zeitschritt berechnet;
    Berechnen (S209, S314) eines Verhaltens eines Mesomodells (13b), das Knotenpunkte (22) hat und das ein Modell der Zelle ist, basierend auf dem berechneten Verhalten des Mikromodells (13c), das in einer biologischen Simulation des Materials verwendet wird, wobei das Berechnen Verschiebungen ($x_{F,e}$) der Knotenpunkte (22) auf der Basis eines Simulationsergebnisses des Mesomodells (13b) zu jedem Zeitschritt berechnet;
    Aktualisieren (S219, S317) des Verhaltens des Mesomodells (13b) der Zelle auf der Basis das berechneten Verhaltens des Makromodells (13a), das in einer biologischen Simulation des Organs verwendet wird, entsprechend den Verschiebungen ($x_c$, $x_{F,e}$) der Knotenpunkte des Makromodells (13a) und des Mesomodells (13b); und
    Aktualisieren (S220, S318) des Verhaltens des Mikromodells (13c) des Materials auf der Basis des aktualisierten Verhaltens des Mesomodells (13b), das in einer biologischen Simulation der Zelle verwendet wird, entsprechend den Verschiebungen ($x_c$, $x_{F,e}$) der Knotenpunkte des Mesomodells (13b) und des Makromodells (13a), wobei das biologische Simulationsprogramm:

        den ersten Prozessor (14b, 24b) veranlasst, das Verhalten des Makromodells (13a) zu berechnen, und den zweiten Prozessor (14a, 24a) veranlasst, das Verhalten des Mikromodels (13c) zu berechnen, die Kraft zu berechnen, die auf Knotenpunkte des Makromodells (13a) ausgeübt wird, das Verhalten des Mesomodells (13b) zu berechnen, das Verhalten des Mesomodells (13b) zu aktualisieren und das Verhalten des Mikromodells (13c) zu aktualisieren.

2. Biologische Simulationsvorrichtung, umfassend:

einen ersten Prozessor (14b, 24a); und

einen zweiten Prozessor (14a, 24a), wobei

der erste Prozessor (14b, 24b) und der zweite Prozessor (14a, 24a) einen Prozess ausführen, umfassend:

durch den zweiten Prozessor (14a, 24a), Berechnen (S207, S307) eines Verhaltens eines Mikromodells (13c), das Knotenpunkte (24) hat und das ein Modell eines Materials ist, welches in einer Zelle eines Organs eines biologischen Körpers enthalten ist, wobei das Organ durch ein Makromodell (13a) modelliert wird, das Knotenpunkte hat, die Berechnung berechnet Verschiebungen ($x_{M,e}$) der Knotenpunkte auf der Basis eines Simulationsergebnisses des Mikromodells (13c) zu jedem Zeitschritt;

durch den zweiten Prozessor (14a, 24a), Berechnen einer Kraft, die auf die Knotenpunkte des Makromodells (13a) ausgeübt wird;

durch den ersten Prozessor (14b, 24b), Berechnen (S213, S310) eines Verhaltens des Makromodells (13a) auf der Basis der berechneten Kraft, die auf die Knotenpunkte des Makromodells (13a) ausgeübt wird, die Berechnung berechnet Verschiebungen ($x_c$) der Knotenpunkte auf der Basis eines Simulationsergebnisses des Makromodells (13a) zu jedem Zeitschritt;

durch den zweiten Prozessor (14a, 24a), Berechnen (S209, S314) eines Verhaltens eines Mesomodells (13b), das Knotenpunkte (22) hat und das ein Modell der Zelle ist, basierend auf dem berechneten Verhalten des Mikromodells (13c), das in einer biologischen Simulation des Materials verwendet wird, wobei das Berechnen Verschiebungen ($x_{F,e}$) der Knotenpunkte des Makromodells (13a) berechnet;

durch den zweiten Prozessor (14a, 24a), Aktualisieren (S219, S317) des Verhaltens des Mesomodells (13b) der Zelle, basierend auf dem berechneten Verhalten des Makromodells (13a), das in einer biologischen Simulation des Organs verwendet wird, entsprechend den Verschiebungen ($x_c$, $x_{F,e}$) der Knotenpunkte des Makromodells (13a) und des Mesomodells (13b); und

durch den zweiten Prozessor (14a, 24a), Aktualisieren (S220, S318) des Verhaltens des Mikromodells (13c) des Materials, basierend auf dem aktualisierten Verhalten des Mesomodells (13b), das in einer biologischen Simulation der Zelle verwendet wird, entsprechend den Verschiebungen ($x_c$, $x_{F,e}$) der Knotenpunkte des Mesomodells (13b) und des Makromodells (13c),

wobei jede Berechnung des Verhaltens des Mikromodells (13c), die Berechnung des Verhaltens des Makromodells (13a), die Berechnung des Verhaltens des Mesomodells (13b), die Aktualisierung des Verhaltens des Mesomodells (13b) und die Aktualisierung des Verhaltens des Mikromodells (13c) durch den ersten Prozessor (14b, 24b) und die Berechnung der Kraft, die auf die Knotenpunkte des Makromodells (13a) ausgeübt wird, durch den zweiten Prozessor (14a, 24a) parallel ausgeführt werden.

## Revendications

1. Programme de simulation biologique qui, lorsqu'il est exécuté par un ordinateur ayant un premier processeur (14b, 24b) et un second processeur (14a, 24a), amène l'ordinateur à exécuter un procédé de simulation biologique comprenant les étapes consistant à :

calculer (S207, S307) un comportement d'un micro-modèle (13c) qui comporte des points nodaux (24) et qui est un modèle d'un matériau contenu dans une cellule d'un organe d'un corps biologique, l'organe étant modélisé par un macro-modèle (13a) qui a des points nodaux, l'étape de calcul calculant des déplacements ($x_{M,e}$) des points nodaux (24) sur la base d'un résultat de simulation du micro-modèle (13c) à chaque pas de temps ;

calculer une force qui est appliquée aux points nodaux du macro-modèle (13a) ;

calculer (S213, S310) un comportement du macro-modèle (13a) sur la base de la force calculée qui est appliquée aux points nodaux du macro-modèle (13a), l'étape de calcul calculant des déplacements ($x_C$) des points nodaux sur la base d'un résultat de simulation du macro-modèle (13a) à chaque pas de temps ;

calculer (S209, S314) un comportement d'un méso-modèle (13b) qui comporte des points nodaux (22) et qui est un modèle de la cellule, sur la base du comportement calculé du micro-modèle (13c) utilisé dans une simulation biologique du matériau, l'étape de calcul calculant des déplacements ($x_{F,e}$) des points nodaux (22) sur la base d'un résultat de simulation du méso-modèle (13b) à chaque pas de temps ;

mettre à jour (S219, S317) le comportement du méso-modèle (13b) de la cellule, sur la base du comportement calculé du macro-modèle (13a) utilisé dans une simulation biologique de l'organe, en fonction de déplacements ($x_C$, $x_{F,e}$) des points nodaux du macro-modèle (13a) et du méso-modèle (13b) ; et

mettre à jour (S220, S318) le comportement du micro-modèle (13c) du matériau, sur la base du comportement mis à jour du méso-modèle (13b) utilisé dans une simulation biologique de la cellule, en fonction de déplacements ($x_C$, $x_{F,e}$) des points nodaux du méso-modèle (13b) et du macro-modèle (13a), dans lequel

le programme de simulation biologique amène :

le premier processeur (14b, 24b) à calculer le comportement du macro-modèle (13a), et
le second processeur (14a, 24a) à calculer le comportement du micro-modèle (13c), calculer la force qui est appliquée aux points nodaux du macro-modèle (13a), calculer le comportement du méso-modèle (13b), mettre à jour le comportement du méso-modèle (13b) et mettre à jour le comportement du micro-modèle (13c).

2. Dispositif de simulation biologique comprenant :

un premier processeur (14b, 24b) ; et
un second processeur (14a, 24a), dans lequel
le premier processeur (14b, 24b) et le second processeur (14a, 24a) exécutent un procédé comprenant les étapes consistant à :

calculer (S207, S307), par l'intermédiaire du second processeur (14a, 24a), le comportement d'un micro-modèle (13c) qui comporte des points nodaux (24) et qui est un modèle d'un matériau contenu dans une cellule d'un organe d'un corps biologique, l'organe étant modélisé par un macro-modèle (13a) qui comporte des points nodaux, l'étape de calcul calculant des déplacements ($x_{M,e}$) des points nodaux sur la base d'un résultat de simulation du micro-modèle (13c) à chaque pas de temps ;
calculer, par l'intermédiaire du second processeur (14a, 24a), une force qui est appliquée aux points nodaux du macro-modèle (13a) ;
calculer (S213, S310), par l'intermédiaire du premier processeur (14b, 24b), un comportement du macro-modèle (13a) sur la base de la force calculée qui est appliquée aux points nodaux du macro-modèle (13a), l'étape de calcul calculant des déplacements ($x_C$) des points nodaux sur la base d'un résultat de simulation du macro-modèle (13a) à chaque pas de temps ;
calculer (S209, S314), par l'intermédiaire du second processeur (14a, 24a), un comportement d'un méso-modèle (13b) comportant des points nodaux (22) et qui est un modèle de la cellule, sur la base du comportement calculé du micro-modèle (13c) utilisé dans une simulation biologique du matériau, l'étape de calcul calculant des déplacements ($x_{F,e}$) des points nodaux du macro-modèle (13a) ;
mettre à jour (S219, S317), par l'intermédiaire du second processeur (14a, 24a), le comportement du méso-modèle (13b) de la cellule, sur la base du comportement calculé du macro-modèle (13a) utilisé dans une simulation biologique de l'organe, en fonction de déplacements ($x_C$, $x_{F,e}$) des points nodaux du macro-modèle (13a) et du méso-modèle (13b) ; et
mettre à jour (S220, S318), par l'intermédiaire du second processeur (14a, 24a), le comportement du micro-modèle (13c) du matériau, sur la base du comportement mis à jour du méso-modèle (13b) utilisé dans une simulation biologique de la cellule, en fonction de déplacements ($x_C$, $x_{F,e}$) des points nodaux du méso-modèle (13b) et du macro-modèle (13c),
dans lequel l'un quelconque du calcul du comportement du micro-modèle (13c), du calcul du comportement du macro-mode (13a), du calcul du comportement du méso-modèle (13b), de la mise à jour du comportement du méso-modèle (13b), et de la mise à jour du comportement du micro-modèle (13c) par le premier processeur (14b, 24b) et le calcul de la force qui est appliquée aux points nodaux du macro-modèle (13a) par le second processeur (14a, 24a) sont exécutés en parallèle.

# FIG.1

BIOLOGICAL SIMULATION DEVICE ⌐10

CONTROLLER ⌐14

⌐11
INPUT UNIT

⌐12
DISPLAY UNIT

MACRO PROCESSOR ⌐14a

MACRO PROCESSOR ⌐14a

⌐15a
CALCULATOR

MESO-MICRO PROCESSOR

MESO-MICRO PROCESSOR ⌐14b

⌐15b
FIRST CALCULATOR

⌐15c
SECOND CALCULATOR ⌐14b

⌐15d
FIRST UPDATING UNIT

⌐15e
SECOND UPDATING UNIT

⌐14c
DISPLAY CONTROLLER

STORAGE UNIT ⌐13

⌐13a
MACRO MODEL

⌐13b
MESO MODEL

⌐13c
MICRO MODEL

⌐13d
CONDITION DATA

# FIG.2

# FIG.3

| TIME STEP | Ca²⁺ CONCENTRATION |
|-----------|--------------------|
| 1 | ......... |
| 2 | ......... |
| ⋮ | ⋮ |

# FIG.4

```
                    ┌──────────────┐
                    │    START     │
                    └──────┬───────┘
                           │              ◄─────────────────────┐
                           ▼                                     │
            ┌──────────────────────────────┐                    │
            │    SIMULATION PROCESSING      │─── S101            │
            └──────────────┬───────────────┘                    │
                           │                                     │
                           ▼      S102                           │
                        ╱────────╲                              │
                       ╱          ╲         NO                   │
                      ╱ IS TIME STEP ╲──────────┐                │
                      ╲ t FINAL TIME ╱          ▼   S103         │
                       ╲  STEP?     ╱      ┌───────────┐         │
                        ╲──────────╱       │  t=t+1    │─────────┘
                           │               └───────────┘
                          YES
                           ▼
            ┌──────────────────────────────┐
            │    PERFORM CONTROL TO         │─── S104
            │  DISPLAY SIMULATION RESULT    │
            └──────────────┬───────────────┘
                           │
                           ▼
                    ┌──────────────┐
                    │     END      │
                    └──────────────┘
```

# FIG.5

**MACRO PROCESSOR**

S201
TRANSMIT Ca$^{2+}$ CONCENTRATION

S204
TRANSMIT NODE INFORMATION

S212
RECEIVE $A_{C,e}$, $f_{C,e}$, $S_{C,e}$, AND $b_{C,e}$

S213
ASSEMBLE MATRICES AND OBTAIN MACRO SOLUTION

S214
CONVERGED?
YES
NO

S215
TRANSMIT MACRO SOLUTION

S216
TRANSMIT MESSAGE INDICATING THAT MACRO SOLUTION HAS CONVERGED

RETURN

**MESO-MICRO PROCESSOR**

S202
RECEIVE Ca$^{2+}$ CONCENTRATION

S203
MONTE CARLO OPERATION

S205
RECEIVE NODE INFORMATION

S206
FORM MICRO EQUATION

S207
OBTAIN MICRO SOLUTION

S208
ASSEMBLE MATRICES

S209
OBTAIN MESO SOLUTION

S210
CONVERGED?
NO
YES

S211
TRANSMIT $A_{C,e}$, $f_{C,e}$, $S_{C,e}$, AND $b_{C,e}$

S217
CONVERGED?
YES
NO

S218
RECEIVE MACRO SOLUTION

S219
UPDATE MESO SOLUTION

S220
UPDATE MICRO SOLUTION

RETURN

# FIG.6A

30

# FIG.6B

31

# FIG.6C

32
32a
32b

# FIG.7

TIME

MACRO PROCESSOR

MESO-MICRO PROCESSOR

(S201)
(S204)
(S212)
(S213)
(S205)
(S206 TO S210)
(S211)
(S203)
(S202)

# FIG.8

BIOLOGICAL SIMULATION DEVICE — 20

CONTROLLER — 24

INPUT UNIT — 11

DISPLAY UNIT — 12

MACRO PROCESSOR — 24a

MACRO PROCESSOR — 24a
CALCULATOR — 25a

MESO-MICRO PROCESSOR

MESO-MICRO PROCESSOR — 24b
FIRST CALCULATOR — 25b
SECOND CALCULATOR — 25c
THIRD CALCULATOR — 25d
FIRST UPDATING UNIT — 25e
SECOND UPDATING UNIT — 25f
— 24b

DISPLAY CONTROLLER — 14c

STORAGE UNIT — 13
MACRO MODEL — 13a
MESO MODEL — 13b
MICRO MODEL — 13c
CONDITION DATA — 13d

21

# FIG.9

```
        ┌─────────────────┐                    ┌─────────────────┐
        │      MACRO       │- - - - - - - - - - │   MESO-MICRO     │
        │    PROCESSOR     │                    │    PROCESSOR     │
        └────────┬────────┘                    └────────┬────────┘
                 │              ⌐S301                    │              ⌐S302
        ┌────────▼────────┐                    ┌────────▼────────┐
        │  TRANSMIT Ca²⁺   │───────────────────▶│   RECEIVE Ca²⁺   │
        │  CONCENTRATION   │                    │  CONCENTRATION   │
        └─────────────────┘                    └────────┬────────┘
                                                        │              ⌐S303
                                               ┌────────▼────────┐
                                               │  MONTE CARLO     │
                                               │  OPERATION       │
                 ┌───────────────────┐         └────────┬────────┘
                 │            ⌐S304   │                  │       ⌐S305
        ┌────────▼────────┐          │         ┌────────▼────────┐
        │  TRANSMIT NODE   │──────────┼────────▶│  RECEIVE NODE   │
        │  INFORMATION     │          │         │  INFORMATION    │
        └─────────────────┘          │         └────────┬────────┘
                                      │                  │       ⌐S306
                                      │         ┌────────▼────────┐
                                      │         │ FORM MICRO EQUATION │
                                      │         └────────┬────────┘
                                      │                  │       ⌐S307
                                      │         ┌────────▼────────┐
                                      │         │ OBTAIN MICRO SOLUTION │
                 ⌐S309                │         └────────┬────────┘
        ┌────────▼────────┐          │                  │       ⌐S308
        │ RECEIVE A_C,e, f_C,e, AND │◀─┼─────────┤ TRANSMIT A_C,e, f_C,e, AND │
        │      S_C,e       │          │         │      S_C,e       │
        └────────┬────────┘          │         └────────┬────────┘
                 │       ⌐S310        │                  │       ⌐S313
        ┌────────▼────────┐          │         ┌────────▼────────┐
        │ ASSEMBLE MATRICES│          │         │ ASSEMBLE MATRICES │
        │ AND OBTAIN MACRO │          │         └────────┬────────┘
        │    SOLUTION      │          │                  │       ⌐S314
        └────────┬────────┘          │         ┌────────▼────────┐
                 │       ⌐S311        │         │ OBTAIN MESO SOLUTION │
        ┌────────▼────────┐  YES      │         └────────┬────────┘
        ◀   CONVERGED?    ▶───────┐   │                  │       ⌐S315
        └────────┬────────┘       │   │         ◀   CONVERGED?   ▶─── YES
              NO │  ⌐S312          │   │         └────────┬────────┘
        ┌────────▼────────┐       │   │              NO │  ⌐S316
        │ TRANSMIT MACRO   │───────┼──┼────────▶│ RECEIVE MACRO   │
        │    SOLUTION      │       │   │         │    SOLUTION     │
        └─────────────────┘       │   │         └────────┬────────┘
                                  │   │                  │       ⌐S317
                                  │   │         ┌────────▼────────┐
                                  │   │         │ UPDATE MESO SOLUTION │
                                  │   │         └────────┬────────┘
                                  │   │                  │       ⌐S318
                                  │   │         ┌────────▼────────┐
                                  │   │         │ UPDATE MICRO    │
                                  │   │         │    SOLUTION     │
                                  │   │         └─────────────────┘
        ┌─────────────────┐       │             ┌─────────────────┐
        │     RETURN       │◀──────┘             │     RETURN       │
        └─────────────────┘                     └─────────────────┘
```

# FIG.10

# FIG.11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007192075 A

### Non-patent literature cited in the description

- **A. HOSOI ; T. WASHIO ; J. OKADA ; Y. KADOOKA ; K. NAKAJIMA ; T. HISADA.** A multi-scale heart simulation on massively parallel computers. *ACM/IEEE Supercomputing Conference (SC10),* 2010